# EUROPEAN PATENT SPECIFICATION

(11) **EP 4 432 965 B1**
(45) Date of publication and mention of the grant of the patent: **18.06.2025**
(21) Application number: 22818249.9
(22) Date of filing: 14.11.2022
(51) Int. Cl.: A61C 5/90, A61B 1/24, A61B 1/00, A61C 17/10

(54) **VERSATILE DENTAL KIT FOR INTRAORAL SCANNING**
VIELSEITIGES ZAHNÄRZTLICHES KIT ZUR INTRAORALEN ABTASTUNG
KIT DENTAIRE POLYVALENT POUR BALAYAGE INTRABUCCAL

(30) Priority: 18.11.2021 IT 202100029168
(43) Date of publication of application: 25.09.2024
(73) Proprietor: Salus Oris S.r.l., 83050 Vallesaccarda (AV) (IT)
(72) Inventor: LO RUSSO, Domenico, 83050 Vallesaccarda (AV) (IT)
(74) Representative: Fiammenghi, Eva
(86) International application number: PCT/EP2022/081827
(87) International publication number: WO 2023/088845

(56) References cited:
- WO-A1-2021/130582
- DE-U1- 202005 000 534
- DE-U1- 202008 009 259
- US-A1- 2004 185 415

## Description

### Field of the invention

The present description relates to the medical field and in particular to the dental field. Even more in detail, the present invention relates to a particular kit of dental instruments to be used for intraoral scanning of the arches, typically but not limited to, edentulous arches of a patient. Said kit is characterized in that it comprises instruments which have structural features such as to make them consistently effective and more durable and less subject to wear and tear than similar pre-existing instruments and of which those of the kit according to the present invention represent an evolution.

### Prior art

To date, removable prostheses used for the rehabilitation of people who are missing some or all of their teeth are built from impressions of the patient's edentulous arches. These impressions are made with various types of materials, which are usually prepared by mixing a base compound and a catalyst; as soon as they are prepared, these impression materials have a pasty consistency and require specific impression trays (instruments designed to contain them) in order to be inserted into the patient's mouth in contact with the arches, where as they harden they take the shape thereof, registering it. From this impression, which is the negative of the three-dimensional shape of the arches, a model is created (which represents the positive, i.e. it has the same shape as the arches). This model is obtained by pouring into the impression a material that is usually a specific plaster, which in turn must solidify in order to be used. This first model cannot be used to construct the prosthesis because it does not have adequate accuracy. This lack of accuracy depends on the fact that the materials used for taking the impression undergo dimensional changes that are proportional to the quantity and thickness of the material used. Since the first impression is taken using a standard impression tray, which as such has medium dimensions in order to be applied to everyone's mouth, which inevitably involves an approximation that requires the use of large quantities of material, especially in non-homogeneous thicknesses; this results in increased dimensional changes and, even worse, inhomogeneous changes in the different areas of the arch, which cause distortions and inaccuracies such as to make the first model, derived from that impression, not sufficiently accurate for the construction of the prosthesis. Therefore, the first model is only used to build an individual tray for that patient, so that a second impression can be taken with a minimum amount of material and in homogeneous thicknesses. All this clearly entails the need to repeat procedures, with obvious negative aspects. From the patient's point of view, repeating the same procedure means having to undergo several operative sessions; in addition to the waste of time, this can be an important problem, especially considering that generally the people who need removable prostheses are elderly people, who live alone and are often not completely self-sufficient and may have difficulty moving from their houses or residential structures that host them in order to reach the dental office.

There may also be discomfort for the patient. Taking the impression is really unwelcome due to the sensation of suffocation that in general it causes and/or to the stimulation of the gag reflex, to which many are particularly sensitive, also in relation to the unpleasant taste of many of the impression materials that require almost five minutes for proper hardening in the patient's mouth. Furthermore, all this adds to problems relating to potential allergy phenomena to the materials used.

Another drawback is represented by the waste of time for the clinician and for the dental laboratory. This negatively affects the efficiency of healthcare facilities and related laboratories, inevitably leading to an increase in costs which ultimately negatively affects the user of the service, the patient, and sometimes affects the access thereof to care.

The waste of materials also has multiple implications, both linked to the waste of resources for the acquisition of these materials, as well as for their management, and as regards their disposal with the consequent environmental impact.

In recent years, new technologies have been introduced in dentistry, some of which allow the shape of the patient's teeth and gums to be detected by means of real intraoral scanners. These intraoral scanners are commonly used to make the fixed dental prosthesis, that is the one anchored to the teeth, but they are not used for the removable prosthesis, especially for the total removable prosthesis (the one for the patient who has no residual teeth). The reason for this lack of use is linked to the fact that, in the situation in which the teeth are missing and in practice only the alveolar arches covered by gingiva and mucosa remain, it is extremely complex to perform an intraoral scan.

In this case, the surface to be scanned (i.e. that of the gingiva and the mucosa that covers the edentulous arches) is in fact devoid of geometric features that can be easily detected by the scanner and, above all, is mobile. In practice, and in an extremely simplistic way, the intraoral scanner detects a series of three-dimensional images of the object to be scanned and combines them one after the other to compose the object; if the object to be scanned (in our case the surface of the edentulous mucosa) moves, it is clear that it is impossible, or in any case extremely inaccurate, to reconstruct a shape that has changed during the scanning process. In order to overcome this difficulty, simplifying and making it possible to obtain the intraoral scan of alveolar arches totally or partially edentulous in a predictable manner, a kit of auxiliary instruments for intraoral scanning has already been developed, proposed by the same inventor of the present document, and described in WO2021130582A1. The kit of instruments described in the aforementioned prior art document allows the following objects to be achieved: Opening the tissues surrounding the edentulous arches, physically moving, independently of the patient's will, the cheeks and the tongue in order to move them away from the arches themselves, exposing them in their entirety, allowing and facilitating the access of the scanner; Stabilizing and keeping the tissues still, avoiding that the cheek and tongue muscles that are inserted near the alveolar ridges, contracting (even with small involuntary movements) may move the mucosa that covers the alveolar arches, effectively preventing or hindering the scanning thereof; Facilitating the removal of saliva and preventing the scanner from fogging; Providing a guide for the scanner, facilitating the constant maintenance of the scanner tip (the one that actually captures the object) at an optimal distance from the surface to be scanned. The capacity of the aforementioned kit of instruments allows achieving the aforementioned objects, which are fundamental to allow a rapid, accurate and predictable intraoral scan of totally or partially edentulous arches, allowing them to be used in various fields and disciplines of dentistry and odontostomatology, and is closely related to the particular conformation of its components. In fact, the kit consists of various instruments, two for the lower arch (one instrument for the right side and one for the left side of the lower arch), and one for the upper arch, designed and optimized to adapt specifically to the anatomy of the respective sites of application and use (i.e. upper and lower arches).

In particular, all the instruments, both those for the upper arch and those for the lower arch, consist of a handle with the shape of a parallelepiped, with two arms applied to one of its ends. The arms differ in shape and size between the various instruments; however, for each instrument, these arms are removable and interchangeable with the arms on the opposite side, as well as with arms of different sizes, necessary for adapting to the individual variability between patients. Ultimately, therefore, each instrument is composed of three parts that need to be assembled at the time of their clinical use. This optimizes the usability of the kit of instruments and substantiates the effectiveness thereof, allowing to solve the clinical problems for which the tools have been developed; however, at the same time, the multiplicity of pieces to compose the final instrument configures some technical problems relating to the efficiency of production, the costs of the instruments, their durability over time. In fact, the different parts of the instruments, in order to guarantee their simple, quick, effective and efficient interfacing over time, require accurate machining with minimal tolerances, which affects production costs. In addition, the interfaces between the various components and the coupling mechanisms are both subject to wear which can limit the life cycle of the instruments themselves. All this clearly constitutes an increase in costs for the end user in order to be able to equip themselves with the necessary tools for a correct intraoral scan of the edentulous arches and for their maintenance. The object of the present invention is to propose a new kit of dental instruments for intraoral scanning of a patient which allows all the aforementioned criticalities associated with similar pre-existing devices to be overcome.

### Description of the invention

The present description relates to a particular kit of instruments for medical use and specifically for dental use, which allows a precise intraoral scan of the edentulous alveolar arches to be made possible. All this in order to create the dental prosthesis with greater precision and also speed up the prosthetic implant procedure in the patient. More specifically, the object of the present invention is a kit of dental instruments optimized and more effective with respect to similar pre-existing devices. Said kit, and specifically each instrument included therein, first of all allows:
- Opening the tissues surrounding the edentulous arches. With the loss of teeth, the bone (alveolar arch) that held these teeth is reabsorbed, reducing in size and moving downwards for the lower arch and upwards for the upper arch. As a result, the cheeks and, for the lower arch, the tongue tend to overlap the edentulous crest and mask it. Therefore, for a correct scanning of the edentulous arches it is necessary to separate, i.e. physically move, independently of the patient's will, the cheeks and the tongue in order to move them away from the arches themselves and expose them in their entirety in order to avoid areas in which the overlapping cheeks and tongue "overshadow" the edentulous arch, preventing it from being fully read and captured by the scanner.
- Stabilizing and holding the tissues in place. As detailed above, a key feature of edentulous arches is that the mucosa covering them is mobile. This is linked to the fact that as the edentulous arches are reabsorbed and reduced in size, the gingiva attached to them is also lost, so that a mobile mucosa remains to cover the edentulous arches, the degree of mobility of which is generally proportional to the reabsorption of the alveolar arches. More in detail, "mobile" means that the muscles of the cheeks and tongue that are inserted close to the alveolar ridges can move the mucosa covering the edentulous alveolar arches by contracting. This means that even small involuntary movements of the patient can, as described above, prevent the scanning of the totally or partially edentulous arches. Therefore, for the purpose of a correct intraoral scanning of the edentulous arches it is imperative to keep the tissues of the arch still and stabilized for the entire duration of the scanning process.
- Facilitating the removal of saliva and preventing the scanner from fogging. During the scanning process, especially in the lower arch, saliva will tend to accumulate; this could compromise the correct scanning of some areas, since current scanners are ultimately based on optical technologies that use the reflection of light that could be affected by the different refractive index of saliva. Therefore, it is a great advantage that excess saliva can be easily removed during the scanning process.
- Providing a guide for the scanner. The possibility of keeping the tip of the scanner (the one that actually captures the object) always at an optimal distance from the surface to be scanned is a great advantage as it facilitates the focusing of the object and therefore the acquisition of the images and consequently their accuracy and processing. For this reason, the instrument object of the present invention is made in such a way as to allow the scanner tip to be placed on its parts and to slide over them as if on a guide capable on the one hand of simplifying the movement of the scanner tip during the scanning process and on the other hand to allow the maintenance of an optimal distance from the surface to be captured. Furthermore, this feature is useful for allowing an automated scan in association with an extraoral device which holds the present guide object and, on the basis of it, simultaneously holds and gives a suitable movement to the scanner.

The instrument kit according to the present invention, with its ability to spread the tissues, stabilize and hold them in place for the duration of the scanning process, facilitate the removal of saliva, guide and allow smoother movement of the scanner tip and hold the latter at an optimal distance from the edentulous crest, allows a rapid, accurate and predictable intraoral scan of the totally or partially edentulous arches to be made possible. Therefore, it can find application in various fields and disciplines of dental prosthesis and odontostomatology; in particular, the preferential areas foresee its use in implants of:
- total removable prosthesis with mucous support;
- partial removable prosthesis with mucous support;
- total removable prosthesis with implant support;
- partial removable prosthesis with implant support;
- prostheses on implants;
- fixed prosthesis on natural teeth with extended edentulous saddles.

Said kit specifically allows the acquisition and documentation of the patient's oral anatomical data for diagnostic, anthropometric or identification purposes. It is also intended to be used in patients with natural dentition in which the involuntary movements of the oral muscles prevent a correct scanning of the dental arches and in patients with natural dentition with poor collaboration (for example only: patients with cognitive impairment of various kinds).

Its use is also contemplated in all possible odontostomatological areas, not expressly mentioned, in which it is necessary to achieve the above objectives for which the proposed instrument can be useful by finding its indication. In detail, for the indications of removable prostheses, the use of the instrument allows the application thereof to them and the intraoral scanning thereof, allowing not having to take the traditional impression, or rather two traditional impressions and the construction of the individual impression tray and two plaster models, as the intraoral scan is itself a sufficiently accurate working model for the construction of the prosthesis.

The object of the present invention, in addition to allowing all the aforementioned advantages to be achieved, is specifically that of favoring the diffusion of intraoral scanning for the rehabilitation of the patient with totally or partially edentulous arches through the use of auxiliary instruments that can support the scanning process, overcoming the technological limitations of other similar instruments.

More in detail, the kit of dental instruments according to the present invention is characterized in that said instruments are each made up of a single body. This feature, that of being a monobloc, allows:
Advantageously, to simplify the production process, containing the costs thereof;
Advantageously, to use different production technologies in order to obtain the most adequate balance between the features and quality of the instruments and the investment necessary to obtain them;
Advantageously, to use different materials, exploiting the specific properties and benefits thereof;
Advantageously, to avoid the creation of interfacing and locking mechanisms between the various parts of the instruments; these interface mechanisms are expensive to create and maintain (both in terms of time and costs) and their absence, in addition to constituting an economic and ergonomic advantage, would improve the usability of the instruments by extending the life cycle thereof, given the absence of parts and/or mechanisms (such as interface and locking ones) subject to wear;
Advantageously, to favor the diffusion of the instruments and to obtain the clinical-practical benefits that their use entails. Given these advantages, the instruments of the kit according to the present invention always and in any case allow the tissues to be spread apart, stabilized and held in place for the duration of the scanning process, facilitating the removal of saliva, guiding and allowing smoother movement of the scanner tip and holding the latter at an optimal distance from the edentulous crest, allowing a rapid, accurate and predictable intraoral scan of the totally or partially edentulous arches to be made possible. Therefore, they can find application in various fields and disciplines, such as those listed above, typical of dentistry and odontostomatology, preferably, but not limited to the rehabilitation of edentulous patients.

### Description of the figures

The present invention will be described in detail hereinafter also with reference to the accompanying figures, in which:
Figure 1 shows the first instrument for the left side lower arch 10 of the patient of the kit according to the present invention. The figure shows a top view thereof.
Figure 2 shows the instrument for the left side lower arch 10 of the patient. The figure shows a front view thereof.
Figure 3 shows the instrument for the left side lower arch 10 of the patient in a lateral view and the divergence angle of the lateral and front arms with respect to the vertical plane. This angle for both arms is variable from 10 to 20 degrees, preferably 15 degrees.
Figure 4 shows a plan view of the instrument for the upper arch 50.
Figure 5 also shows the instrument for the upper arch 50 of the patient. The figure shows a front view thereof.
Figure 6 shows the instrument for the upper arch 50. The figure shows in particular the lateral view and the divergence angle of the lateral arms with respect to the vertical plane. This angle for both arms is variable from 20 to 30 degrees, preferably 25 degrees.
Figure 7 shows the development (by unrolling) on plane of the instrument for the lower arch 100 of the patient in a particular embodiment of the kit according to the present invention.
Figure 8 shows the development (by unrolling) on plane of the instrument for the upper arch 500 in a particular embodiment according to the present invention.
Figure 9 shows the instrument for the left side lower arch 100 of the patient of the kit according to the present invention. The figure shows a top view thereof.
Figure 10 shows the instrument for the left side lower arch 100 of the patient. The figure shows a front view thereof in more detail.
Figure 11 also shows the instrument for the left side lower arch 100 of the patient. The figure shows a side view and in particular the divergence angle of the lateral and front arms with respect to the vertical plane. This angle for both arms is variable from 10 to 20 degrees, preferably 15 degrees.
Figure 12 shows a plan view of the instrument for the upper arch 500. The figure shows the top view thereof.
Figure 13 shows the instrument for the upper arch 500. The figure shows the front view thereof.
Figure 14 shows the instrument for the upper arch 500. The figure shows the lateral view thereof and the divergence angle of the lateral arms with respect to the vertical plane. This angle for both arms is variable from 20 to 30 degrees, preferably 25 degrees.
Figure 15 shows the instrument for the left side lower arch 1000 obtained by bending/molding of metal wire and in particular a perspective view thereof. It should be noted that the front arm of the instruments for the lower arch can be simplified by means of a grid-like folding.
Figure 16 shows the instrument for the left side lower arch 1000 obtained by wire bending/molding. The figure shows a top view thereof.
Figure 17 shows the instrument for the lower arch 1000 obtained by bending/molding of metal wire. The figure shows a front view thereof.
Figure 18 shows the instrument for the left side lower arch 1000 obtained by wire bending/molding. The figure shows a side view thereof.
Figure 19 shows the instrument for the upper arch 5000 obtained by wire bending/molding: perspective view.
Figure 20 shows a plan view of the instrument for the upper arch 5000. The figure shows the top view thereof.
Figure 21 shows the instrument for the upper arch 5000. The figure shows a front view thereof.

### Detailed description of the invention

In all its embodiments, the kit 1 of dental instruments according to the present invention is such that its instruments have an optimized and specific shape and structure for the arches.

In particular, said kit 1 comprises at least one instrument for the upper arch, at least one instrument for the right side lower arch and at least one for the left side lower arch; said instruments for the lower arch are one the mirror copy of the other.

In general, although each instrument consists of a single body, which is an aspect that characterizes the kit according to the present invention, it may be schematized herein, only for explanatory purposes and to make the understanding of the invention clearer, in different sections: a body and two arms; for the instruments of the lower arch, the arms are arranged, with respect to the handle, one in the lateral direction and one in the front direction; for the instrument of the upper arch, the arms are structurally mirrored and arranged, with respect to the handle, one in the right lateral direction and the other in the left lateral direction. This general scheme translates into diversified and optimized shapes of the instruments also in relation to the production technology and the material with which they are made.

Below are some embodiments of the kit which correspond to possible solutions, contemplated by the present invention, for obtaining "monobloc" instruments, an aspect which, as repeated many times, characterizes the kit in question.

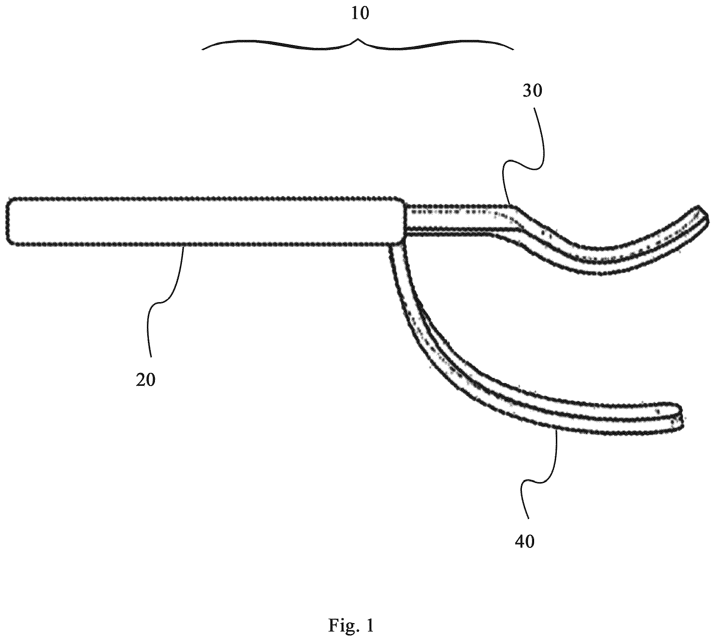

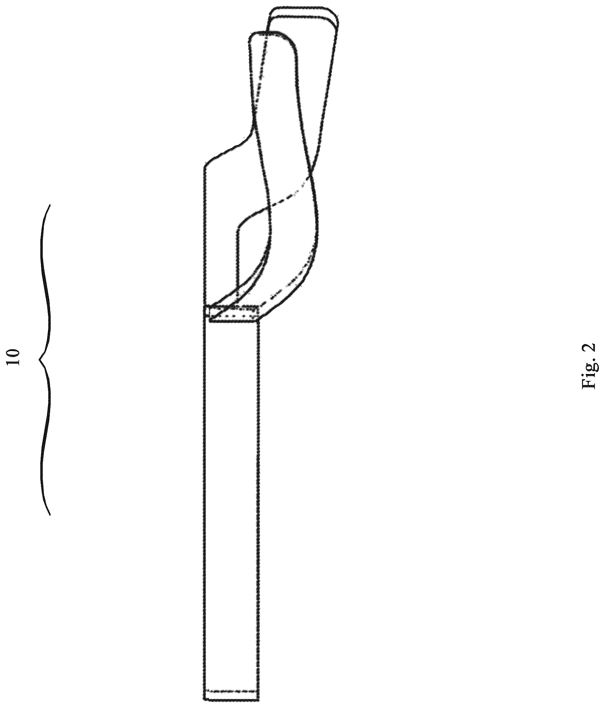

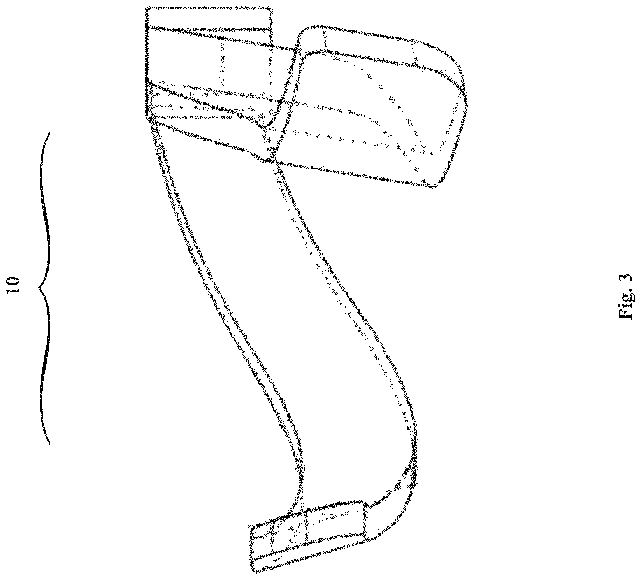

More in detail, in a first embodiment, the kit 1 of dental instruments is such that its instruments may be obtained with known techniques of injection molding of polymeric material and/or molding/bending of metal material with possible welding of the components. Using this technique, the kit 1 in question typically comprises at least one instrument for the left side lower arch 10 of the patient, its mirrored counterpart, i.e. at least one instrument for the right side lower arch of the patient; and at least one instrument for the upper arch 50 of the patient. Said instrument for the left side lower arch 10 of the patient (as well as its mirrored counterpart for the right side) comprises in turn a first lower handle 20, a first front lower arm 30 and a first left lateral lower arm 40. Said instrument for the upper arch 50 of the patient comprises: a first upper handle 60 and two upper lateral arms 70. In this embodiment, said instrument for the left side lower arch 10 (and also the right side) of the patient and said instrument for the upper arch 50 of the patient are such that their handles, i.e. the first lower handle 20 and first upper handle 60 typically but without limitation have the shape of parallelepiped with slightly rounded edges. In particular, with reference to Figures 1, 2, 3 which represent various views of the instrument for the left side lower arch 10 of the patient, two arms can be found at the end of said first lower handle 20, the lower left (or right) lateral one 40 facing outwards which serves to spread the cheek and on which the body of the scanner tip can be placed and made to slide, and the other (the front lower arm 30) facing forward as if to be the extension of the handle that is used to hold and control the tongue and at the same time to control the scanner tip.

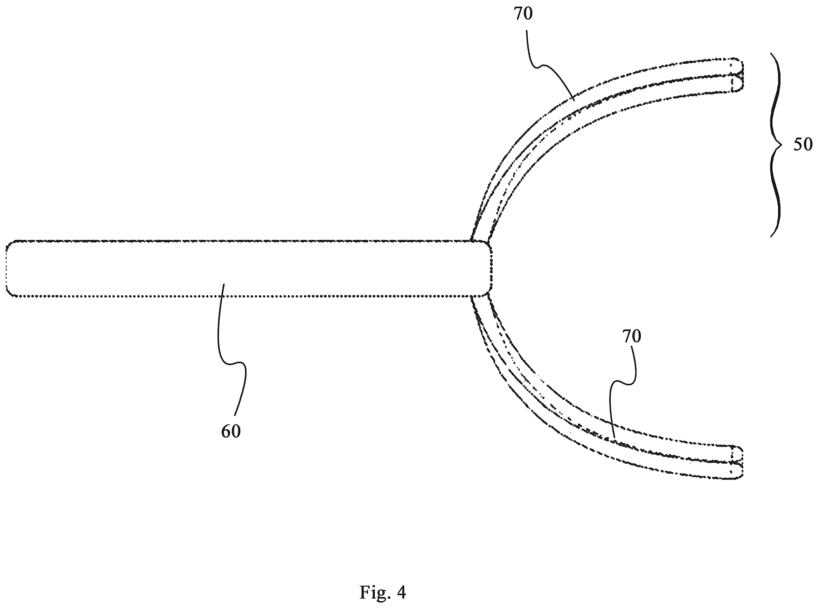

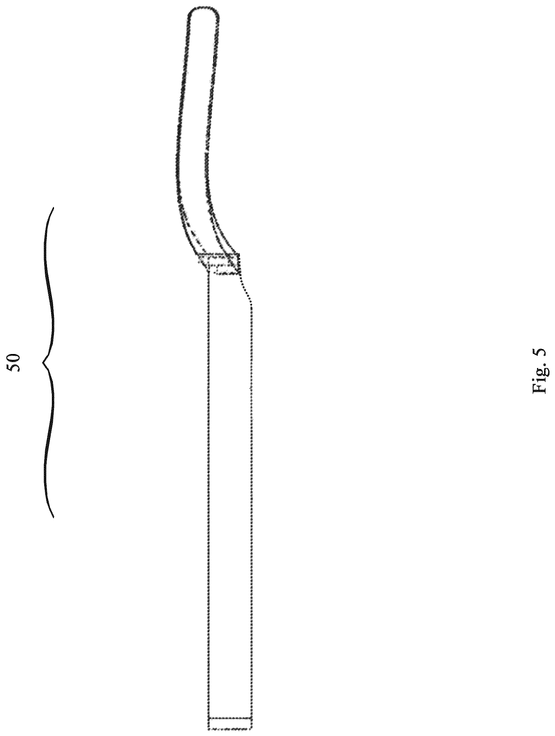

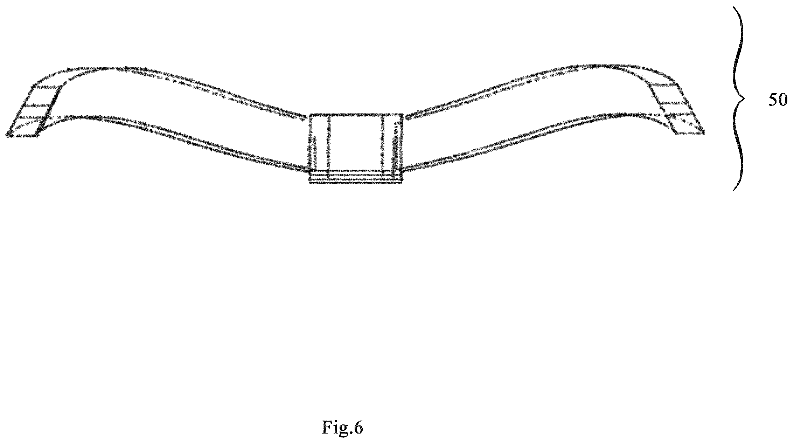

With reference to Figures 4, 5, 6 it can be seen that also the auxiliary instrument for intraoral scanning of the edentulous upper arch, i.e. said instrument for the upper arch 50 of the patient, consists of a handle, i.e. said first upper handle 60 with the shape, preferably, of a parallelepiped with slightly rounded edges, at one of the ends of which there are two arms, both, i.e. said upper lateral arms 70, facing outwards, one towards the right, other to the left, serving to spread apart
the cheeks and on which the body of the scanner tip can be placed and slid.

In this embodiment, said instruments, instrument for the lower arch 10 and instrument for the upper arch 50 described above are characterized in that they consist of a single body, and can be made, by way of non-limiting example, starting from:
- a plastic or polymeric material, which is injected into a mold made to give them the aforementioned final shape;
- a metallic material worked with 3D printing technologies;
- a metal material processed with milling technology from solid blocks;
- starting from pieces of metal material, made separately using appropriate production technologies and subsequently joined and made integral by welding to form a single body.

In a further embodiment, the components of the kit 1 of dental instruments according to the present invention may be obtained by known techniques of (cold or hot) molding and/or bending of sheet steel or other metal.

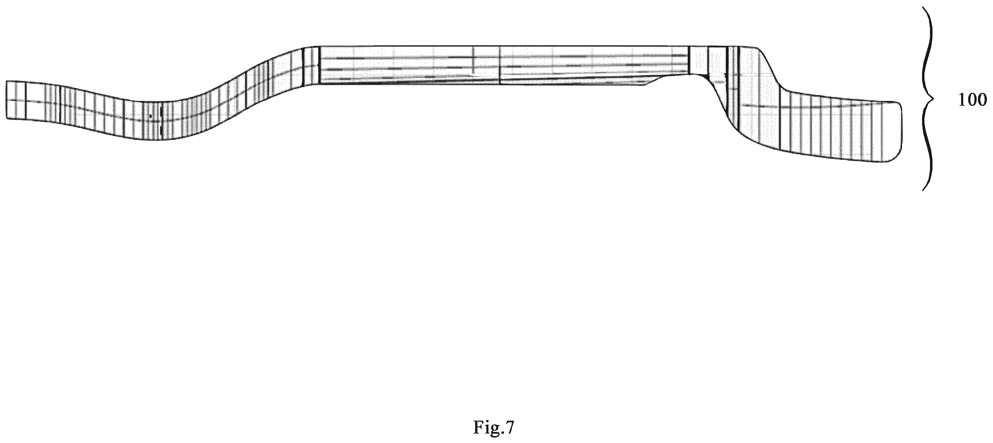

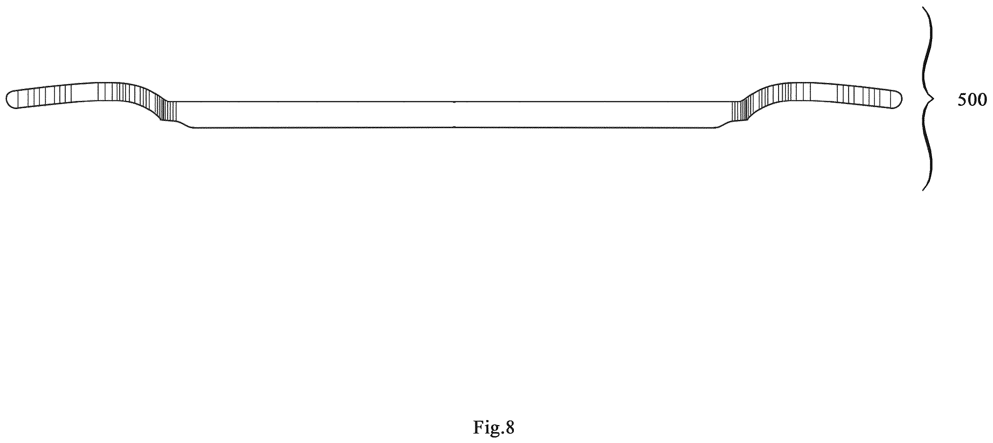

This production method allows obtaining instruments with a shape similar to those described above starting from a piece of sheet metal (solid or perforated) of steel or other metal, by way of non-limiting example titanium, with a thickness of between 0.5 and 1.2 mm, preferably 0.8 mm, the shape of which represents the flat development of said objects, as shown in Figures 7 and 8, for the lower and upper arch, respectively.

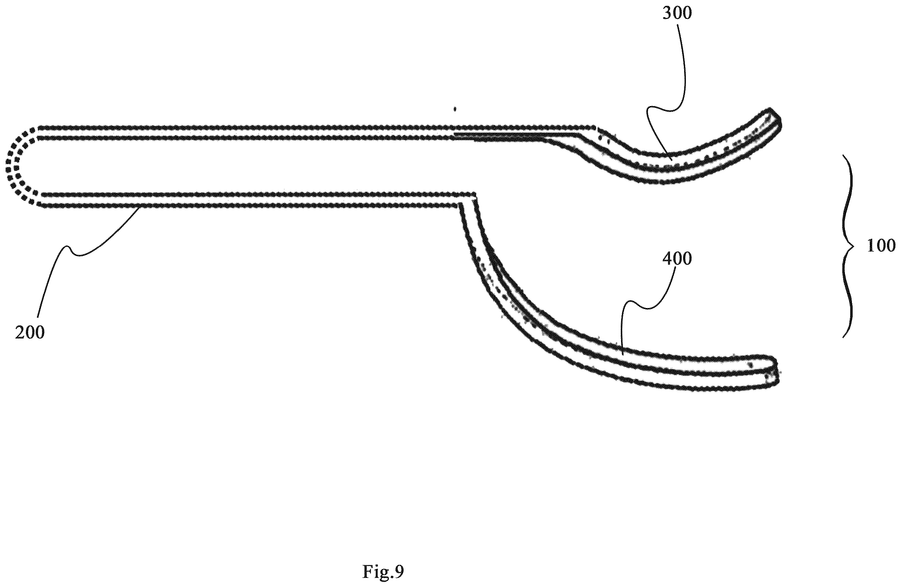

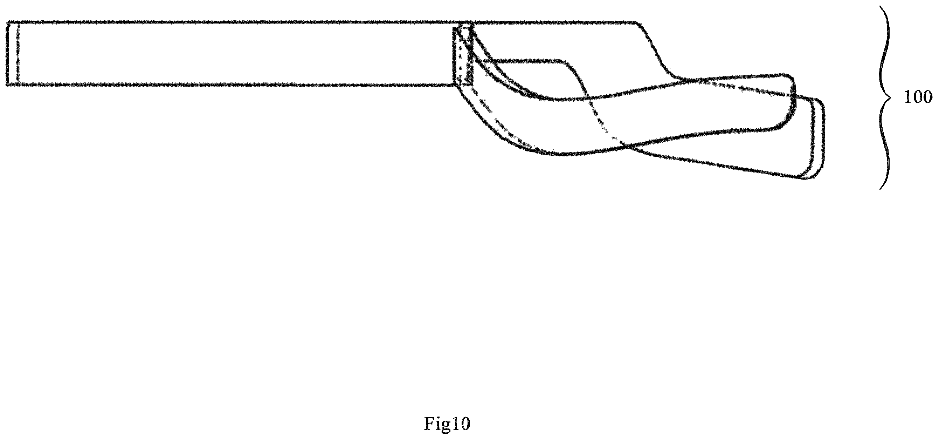

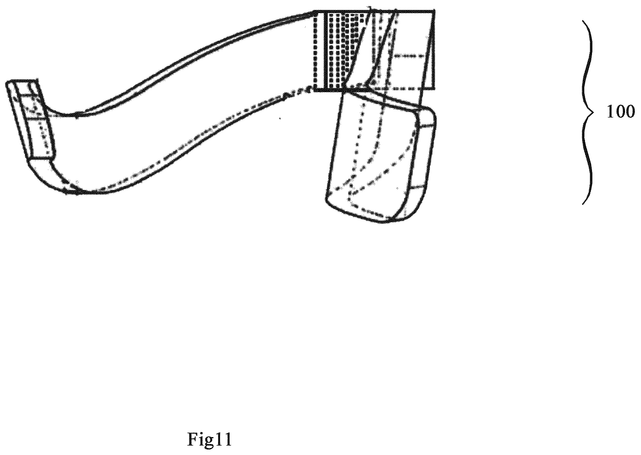

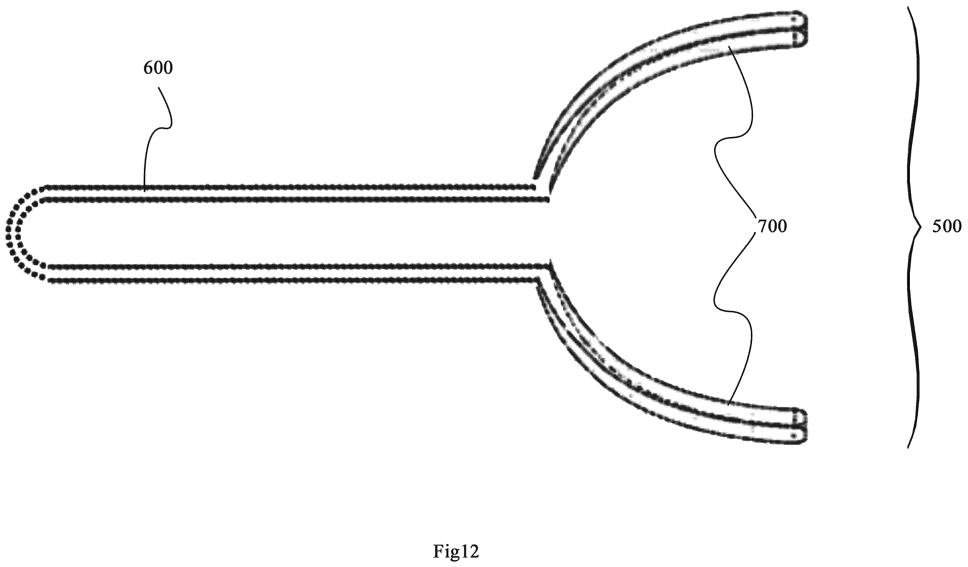

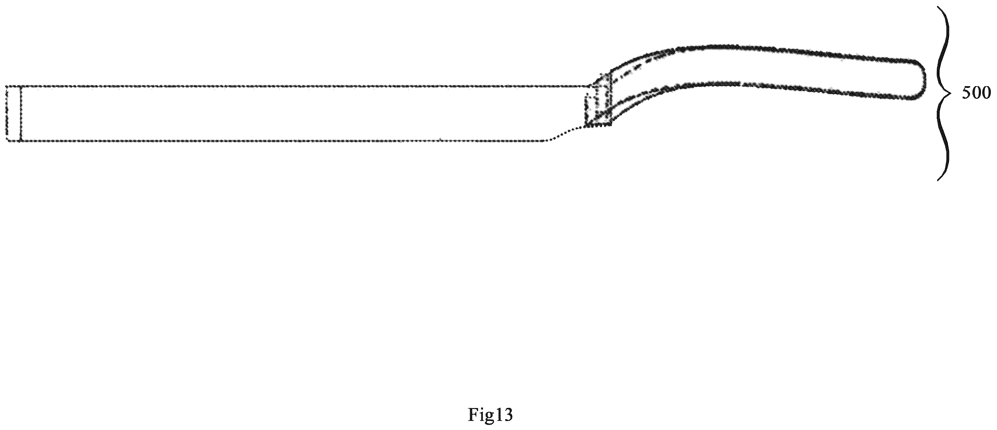

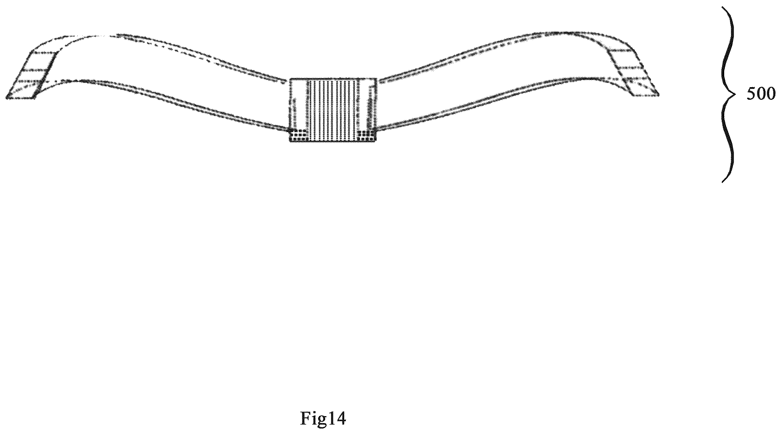

Starting from said sheets of specific shape, as illustrated in Figures 7 and 8, the final instruments (similar in spatial configuration to those described above) are made by means of the action of single or progressive dies, possibly combined with appropriate bending so as to obtain the three-dimensional configuration shown in Figures 9, 10, 11, 12, 13, 14.

In said embodiment, said kit 1 therefore comprises at least one instrument for the left side lower arch 100 of the patient, at least its mirrored counterpart, i.e. at least one instrument for the right side lower arch of the patient; and at least one instrument for the upper arch 500 of the patient. Said instrument for the left side lower arch 100 of the patient (as well as its mirrored counterpart for the right side) comprises in turn a first lower handle 200, a first front lower arm 300 and a first left lateral lower arm 400. Said instrument for the upper arch 500 of the patient comprises: a first upper handle 600 and two upper lateral arms 700. In this embodiment, each of the said instruments for the left (right) side lower arch 100 (right) of the patient and the instrument for the upper arch 500, as well as their arms, are represented by a single piece of curved sheet metal which overall assumes the profile analogous to that described in the aforementioned first embodiment.

In a still further embodiment according to the present invention, the components of the kit of dental instruments according to the present invention are made by means of a known technique of bending steel wire or other metals, by way of non-limiting example titanium.

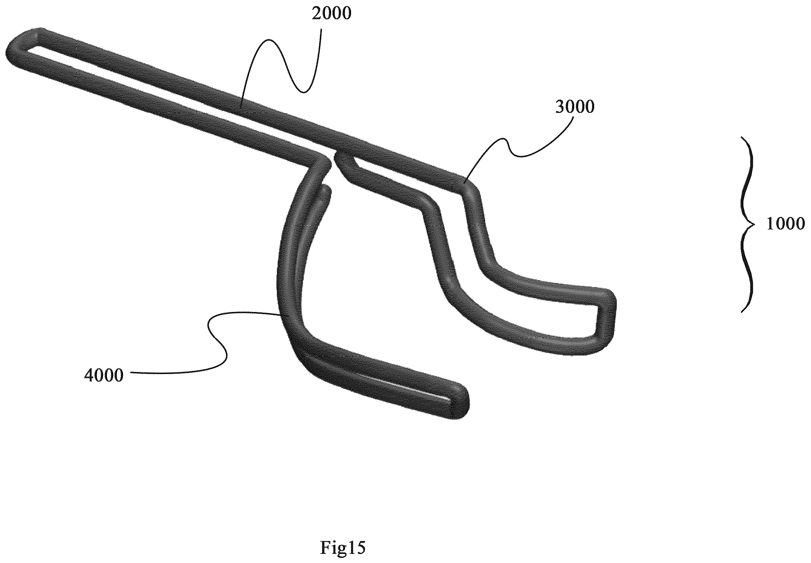

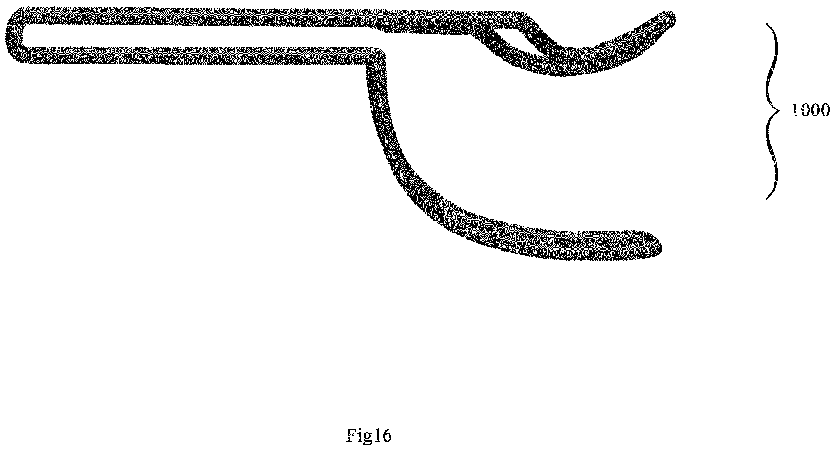

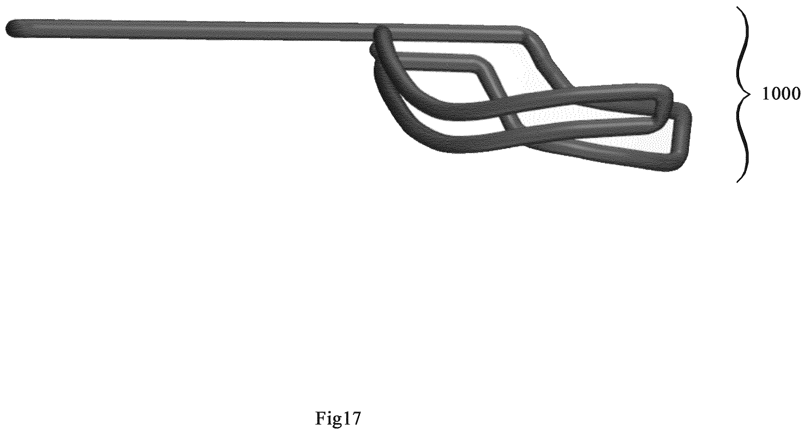

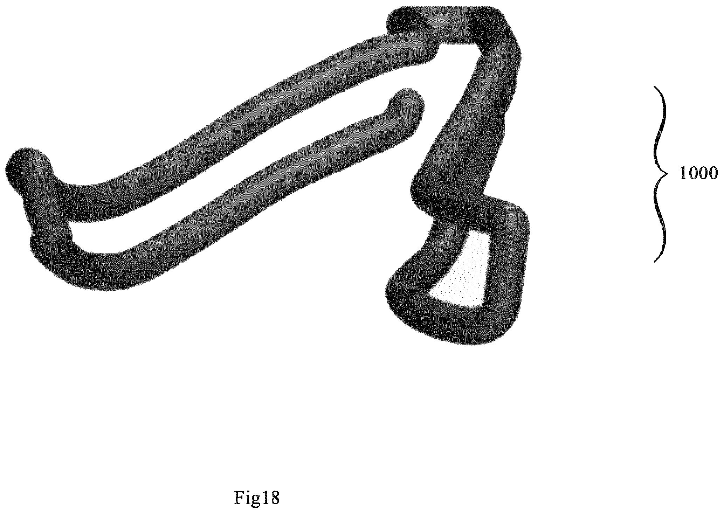

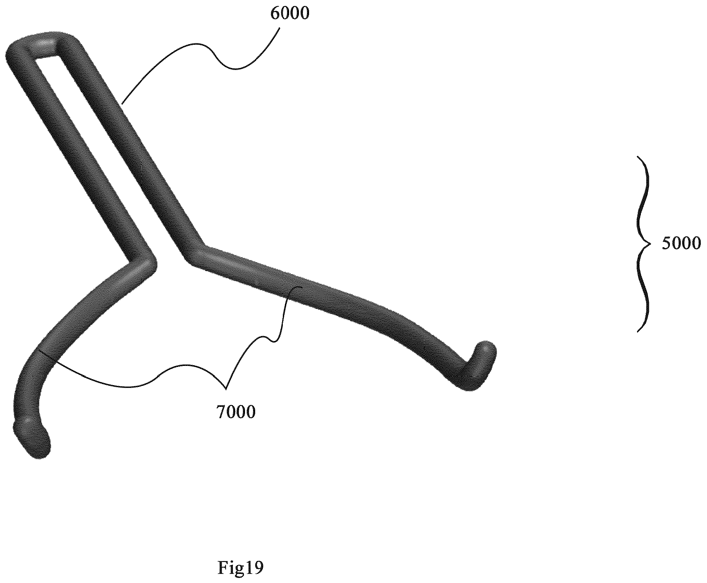

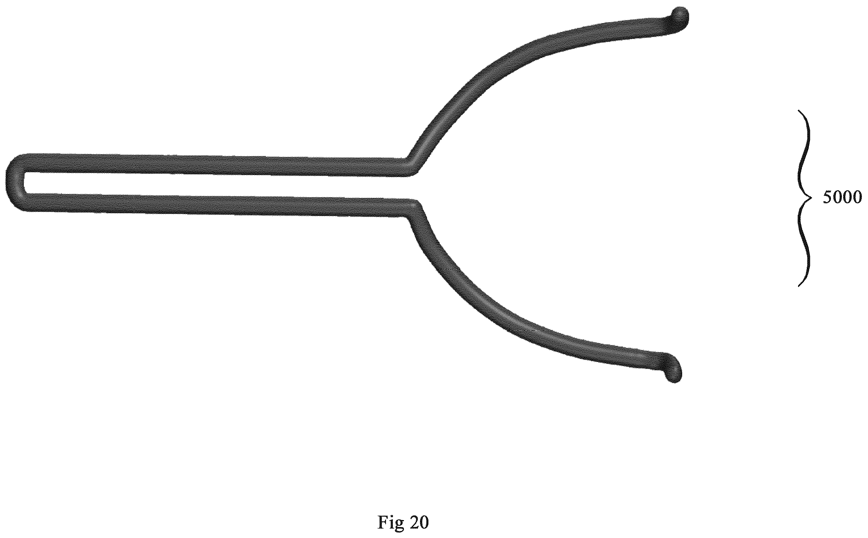

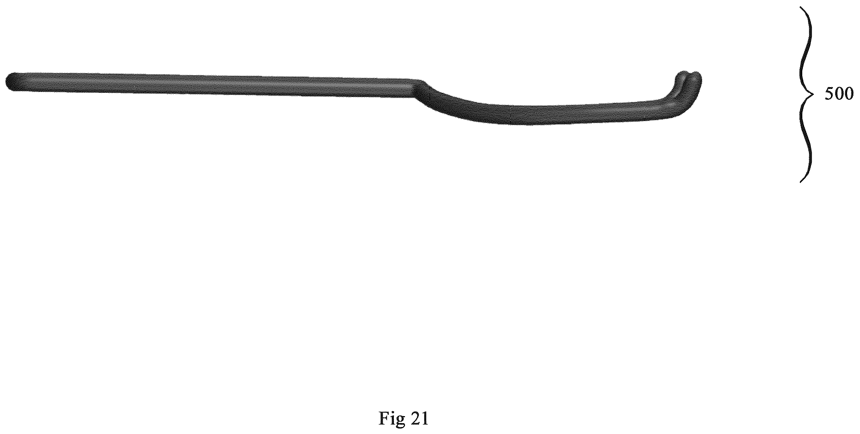

This production method allows obtaining instruments with a shape similar to those described in the aforementioned embodiments starting from steel or other metal wire, with a thickness of between 2 and 5 mm, preferably 3.8 mm, suitably bent and shaped so as to obtain the three-dimensional configuration shown in Figures 15, 16, 17, 18, 19, 20, 21. In detail, the bending and modeling can be carried out, manually or with digitally controlled machines, as if the aforementioned wire were stretched along the edges of the instruments described as in the first embodiment described above, as if to trace the edges thereof.

It should be noted that by means of this method, the above instruments can be made in a single body, both through a single production step, and through the manufacture of pieces (for example, front arm, side, handle or parts of them) which, after being made separately, are subsequently joined and made integral by welding to form a single body.

In such embodiment, the kit of dental instruments according to the present invention therefore comprises at least one instrument for the left side lower arch 1000 of the patient, at least its mirrored counterpart, i.e. at least one instrument for the right side lower arch of the patient; and at least one instrument for the upper arch 5000 of the patient. Said instrument for the left side lower arch 1000 of the patient (as well as its mirrored counterpart for the right side) comprises in turn a first lower handle 2000, a first front lower arm 3000 and a first left lateral lower arm 4000. Said instrument for the upper arch 5000 of the patient comprises: a first upper handle 6000 and two upper lateral arms 7000. In this embodiment, each of said instruments for the left (right) side lower arch 1000 of the patient and the instrument for the upper arch 5000 of the patient, as well as their arms, are represented by a single piece and specifically by a single curved wire of metallic and/or polymeric material, preferably of steel, or titanium, assuming a profile similar to that described in the previous embodiments.

Any combination of the described production methods can be used to obtain the instruments object of the present invention. Therefore, the final shape of the instruments, in relation to the different sections that constitute them (i.e. handle, lateral arm, front arm) will assume that of the described production process.

Preferably, but without limitation, in all its embodiments in said kit of dental instruments it is such that its instruments have the following dimensions: said first instrument for the left side lower arch of the patient and second instrument for the right side lower arch of the patient have a total length of between 150 mm and 155 mm, preferably 152 mm; the left or right side arm has a length of between 55 and 71 mm, preferably 63 mm; the front arm for the left or right side has a length of between 62 and 70 mm, preferably 66 mm. The arms of the instrument for the upper arch have a length comprised between 45 and 60 mm, preferably 55 mm, and the distance between their free end is comprised between 55 and 70 mm, preferably 65 mm.

## Claims

1. Kit (1) of dental instruments for intraoral scanning, said kit comprising at least an instrument for the left side lower arch (10, 100,1000) of the patient, at least an instrument for the right side lower arch of the patient, the latter being structurally mirrored with respect to said instrument for the left side lower arch of the patient, at least an instrument for the upper arch (50, 500, 5000) of the patient, said instruments each having one handle and two arms, said instruments for the lower arch each having a curved arm directed in lateral direction with respect to the handle and one curved arm directed in front direction, said instrument for the upper arch having the arms with one configured in right lateral direction and the other in left lateral direction, said lateral arms being adapted to open the cheeks wide and allow the sliding of a scanner on their body, said kit (1) of dental instruments being **characterized in that** each of said instruments comprised therein is represented by a single body, meaning a monobloc.

2. Kit (1) of dental instruments according to the preceding claim wherein at least an instrument is comprised for the left side lower arch (10) of the patient, as well as its mirrored counterpart, the latter being at least an instrument for the right side lower arch of the patient; and at least an instrument for the upper arch (50) of the patient is also comprised, said instrument for the left side lower arch (10) of the patient and respectively its mirrored counterpart for the right side lower arch of the patient each comprising a first lower handle (20), a first front lower arm (30) and a first left lateral (40) and respectively right lateral lower arm, said instrument for the upper arch (50) of the patient comprising: a first upper handle (60) and two upper lateral arms (70), said instrument for the left side lower arch (10), and right side lower arch, of the patient and said instrument for the upper arch (50) of the patient being such that their handles, i.e. said first lower handle (20) and first upper handle (60) having profile shaped as a parallelepiped with rounded edges, said instruments of the kit each being represented by a single body, meaning a monobloc, in which the arms and the handle are represented by a monobloc of polymer material.

3. Kit (1) of dental instruments according to claim 1 wherein at least an instrument for the left side lower arch (10) of the patient is comprised as well as its mirrored counterpart, the latter being at least an instrument for the right side lower arch of the patient; and at least an instrument for the upper arch (50) of the patient, said instrument for the left side lower arch (10) of the patient and respectively its mirrored counterpart for the right side lower arch of the patient each comprising a first lower handle (20), a first front lower arm (30) and a first left lateral (40) and respectively right lateral lower arm, said instrument for the upper arch (50) of the patient comprising: a first upper handle (60) and two upper lateral arms (70), said instrument for the left side lower arch (10), and right side lower arch, of the patient and said instrument for the upper arch (50) of the patient being such that their handles, i.e. said first lower handle (20) and first upper handle (60) having profile shaped as a parallelepiped with rounded edges, said instruments of the kit each being represented by a single body, meaning a monobloc, wherein the arms and the handle are joined via welding.

4. Kit (1) of dental instruments according to claim 1 wherein at least an instrument for the left side lower arch (100) of the patient is comprised as well as its mirrored counterpart, i.e. at least an instrument for the right side lower arch of the patient; and at least an instrument for the upper arch (500) of the patient, said instrument for the left side lower arch (100) of the patient and respectively its mirrored counterpart for the right side lower arch comprising a first lower handle (200), a first front lower arm (300) and a first left lateral (400) and respectively right lateral lower arm, said instrument for the upper arch (500) of the patient comprising a first upper handle (600) and two upper lateral arms (700), said instruments for the left side and right side lower arch (100) of the patient and said instrument for the upper arch (500) being represented by a single piece, meaning a monobloc, of curved plate, solid or perforated.

5. Kit (1) of dental instruments according to the preceding claim **wherein** the plate is made of a metal, said plate having thickness comprised between 0.5 mm and 1.2 mm.

6. Kit (1) of dental instruments according to the preceding claim **wherein** the plate has thickness of 0.8 mm.

7. Kit (1) according to any one of the claims 4 and 6 **wherein** the plate is as desired made of titanium or steel.

8. Kit (1) of dental instruments according to claim 1 **wherein** at least an instrument for the left side lower arch (1000) of the patient is comprised as well as its mirrored counterpart, i.e. at least an instrument for the right side lower arch of the patient; and at least an instrument for the upper arch (5000) of the patient is also comprised, said instrument for the left side lower arch (1000) of the patient, and respectively its mirrored counterpart for the right side, comprising a first lower handle (2000), a first front lower arm (3000) and a first left lateral (4000) and respectively right lateral lower arm, said instrument for the upper arch (5000) of the patient comprising a first upper handle (6000) and two upper lateral arms (7000), each of said instruments for the left side lower arch (1000) and that for the right side lower arch of the patient and the instrument for the upper arch (5000) of the patient being represented by a single wire of metallic and/or polymer material.

9. Kit (1) of dental instruments according to the preceding claim **wherein** the instruments of the kit are made of titanium or steel.

10. Kit (1) of dental instruments according to any one of the claims 8 and 9 **wherein** the thickness of the wire with which the instruments of the kit are attained is comprised between 2 mm and 5 mm.

11. Kit (1) of dental instruments according to the preceding claim **wherein** the thickness with which the instruments of said kit are attained is 3.8 mm.

12. Kit (1) of dental instruments according to any one of the preceding claims wherein said first instrument for the left side lower arch of the patient and second instrument for the right side lower arch of the patient have total length comprised between 150 mm and 155 mm; the left lateral arm, or right lateral arm of the instruments for the lower arch of the patient (10, 100, 1000) have length comprised between 55 and 71 mm; the front arm (30, 300, 3000) for left side or right side have length comprised between 62 and 70 mm; the arms of the instrument for the upper arch (50, 500, 5000) have length comprised between 45 and 60 mm, and the distance between their free end is comprised between 55 and 70 mm.

13. Kit (1) of dental instruments according to the preceding claim **wherein** the left lateral arm, or right lateral arm of the instruments for the lower arch of the patient (10, 100, 1000) have length of 63 mm; the front arm (30, 300, 3000) for the left side or right side have length of 66 mm; the arms of the instrument for the upper arch (50, 500, 5000) have length of 55 mm, and the distance between their free end is 65 mm.

## Patentansprüche

1. Kit (1) zahnärztlicher Instrumente zur intraoralen Abtastung, wobei das Kit zumindest ein Instrument für den linken Unterkiefer (10, 100, 1000) des Patienten, zumindest ein Instrument für den rechten Unterkiefer des Patienten, wobei letzteres in Bezug auf das Instrument für den linken Unterkiefer des Patienten gespiegelt aufgebaut ist, zumindest ein Instrument für den Oberkiefer (50, 500, 5000) des Patienten, aufweist, wobei die Instrumente jeweils einen Griff und zwei Arme aufweisen, wobei die Instrumente für den Unterkiefer jeweils einen in seitlicher Richtung in Bezug auf den Griff gerichteten gekrümmten Arm und einen nach vorne gerichteten gekrümmten Arm aufweisen, wobei das Instrument für den Oberkiefer die Arme mit einem in rechter seitlicher Richtung und dem anderen in linker seitlicher Richtung ausgebildeten Arm aufweist, wobei die seitlichen Arme dazu ausgelegt sind, die Wangen weit zu öffnen und das Gleiten eines Scanners auf ihrem Körper zu ermöglichen, wobei der Satz (1) zahnärztlicher Instrumente **dadurch gekennzeichnet ist, dass** jedes der darin enthaltenen Instrumente durch einen einzigen Körper, d. h. einen Monoblock, dargestellt ist.

2. Kit (1) zahnärztlicher Instrumente gemäß dem vorangehenden Anspruch, wobei zumindest ein Instrument für den linken Unterkiefer (10) des Patienten sowie dessen gespiegeltes Gegenstück, wobei das Letztere zumindest ein Instrument für den rechten Unterkiefer des Patienten ist, enthalten sind; und auch zumindest ein Instrument für den Oberkiefer (50) des Patienten enthalten ist, wobei das Instrument für den linken Unterkiefer (10) des Patienten und sein gespiegeltes Gegenstück für den rechten Unterkiefer des Patienten jeweils einen ersten unteren Griff (20), einen ersten vorderen unteren Arm (30) und einen ersten linken seitlichen (40) bzw. rechten seitlichen unteren Arm aufweisen, wobei das Instrument für den Oberkiefer (50) des Patienten aufweist: einen ersten oberen Griff (60) und zwei obere seitliche Arme (70), wobei das Instrument für den linken Unterkiefer (10) und den rechten Unterkiefer des Patienten und das Instrument für den Oberkiefer (50) des Patienten so ausgebildet sind, dass ihre Griffe, d. h. der erste untere Griff (20) und der erste obere Griff (60), ein als Parallelepiped geformtes Profil mit abgerundeten Kanten aufweisen, wobei die Instrumente des Kits jeweils durch einen einzigen Körper, d. h. einen Monoblock, in dem die Arme und der Griff durch einen Monoblock aus Polymermaterial verkörpert sind, verkörpert sind.

3. Kit (1) zahnärztlicher Instrumente gemäß Anspruch 1, wobei zumindest ein Instrument für den linken Unterkiefer (10) des Patienten sowie dessen gespiegeltes Gegenstück, wobei das Letztere zumindest ein Instrument für den rechten Unterkiefer des Patienten ist, enthalten sind; und zumindest ein Instrument für den Oberkiefer (50) des Patienten, wobei das Instrument für den linken Unterkiefer (10) des Patienten und dessen gespiegeltes Gegenstück für den rechten Unterkiefer des Patienten jeweils einen ersten unteren Griff (20), einen ersten vorderen unteren Arm (30) und einen ersten linken seitlichen (40) bzw. rechten seitlichen unteren Arm aufweisen, wobei das Instrument für den Oberkiefer (50) des Patienten aufweist: einen ersten oberen Griff (60) und zwei obere seitliche Arme (70), wobei das Instrument für den linken Unterkiefer (10) und den rechten Unterkiefer des Patienten und das Instrument für den Oberkiefer (50) des Patienten so ausgebildet sind, dass ihre Griffe, d. h. der erste untere Griff (20) und der erste obere Griff (60), ein als Parallelepiped geformtes Profil mit abgerundeten Kanten aufweisen, wobei die Instrumente des Kits jeweils durch einen einzigen Körper, d. h. einen Monoblock, verkörpert sind<, wobei die Arme und der Griff durch Schweißen verbunden sind.

4. Kit (1) zahnärztlicher Instrumente gemäß Anspruch 1, wobei zumindest ein Instrument für den linken Unterkiefer (100) des Patienten sowie dessen gespiegeltes Gegenstück, d. h. zumindest ein Instrument für den rechten Unterkiefer des Patienten, enthalten sind; und zumindest ein Instrument für den Oberkiefer (500) des Patienten, wobei das Instrument für den linken Unterkiefer (100) des Patienten und dessen gespiegeltes Gegenstück für den rechten Unterkiefer einen ersten unteren Griff (200), einen ersten vorderen unteren Arm (300) und einen ersten linken seitlichen (400) bzw. rechten seitlichen unteren Arm aufweisen, wobei das Instrument für den Oberkiefer (500) des Patienten einen ersten oberen Griff (600) und zwei obere seitliche Arme (700) aufweist, wobei die Instrumente für den linken und rechten Unterkiefer (100) des Patienten und das Instrument für den Oberkiefer (500) durch ein einziges Stück, d. h. einen Monoblock, aus einer gekrümmten, massiven oder perforierten Platte gebildet sind.

5. Kit (1) zahnärztlicher Instrumente gemäß dem vorangehenden Anspruch, **wobei** die Platte aus einem Metall besteht, wobei die Platte eine Dicke zwischen 0,5 mm und 1,2 mm aufweist.

6. Kit (1) zahnärztlicher Instrumente gemäß dem vorangehenden Anspruch, **wobei** die Platte eine Dicke von 0,8 mm aufweist.

7. Kit (1) gemäß einem der Ansprüche 4 und 6, **wobei** die Platte wahlweise aus Titan oder Stahl hergestellt ist.

8. Kit (1) zahnärztlicher Instrumente gemäß Anspruch 1, **wobei** zumindest ein Instrument für den linken Unterkiefer (1000) des Patienten sowie dessen gespiegeltes Gegenstück, d. h. zumindest ein Instrument für den rechten Unterkiefer des Patienten, enthalten sind; und auch zumindest ein Instrument für den Oberkiefer (5000) des Patienten enthalten ist, wobei das Instrument für den linken Unterkiefer (1000) des Patienten und dessen gespiegeltes Gegenstück für die rechte Seite einen ersten unteren Griff (2000), einen ersten vorderen unteren Arm (3000) und einen ersten linken seitlichen (4000) bzw. rechten seitlichen unteren Arm aufweisen, wobei das Instrument für den Oberkiefer (5000) des Patienten einen ersten oberen Griff (6000) und zwei obere seitliche Arme (7000) aufweist, wobei jedes der Instrumente für den linken Unterkiefer (1000) und das für den rechten Unterkiefer des Patienten und das Instrument für den Oberkiefer (5000) des Patienten durch einen einzigen Draht aus einem metallischen und/oder polymeren Material verkörpert sind.

9. Kit (1) zahnärztlicher Instrumente gemäß dem vorangehenden Anspruch, **wobei** die Instrumente des Kits aus Titan oder Stahl hergestellt sind.

10. Kit (1) zahnärztlicher Instrumente gemäß einem der Ansprüche 8 und 9, **wobei** die Dicke des Drahtes, mit dem die Instrumente des Kits erhalten werden, zwischen 2 mm und 5 mm liegt.

11. Kit (1) zahnärztlicher Instrumente gemäß dem vorangehenden Anspruch, **wobei** die Dicke, mit der die Instrumente des Kits erhalten werden, 3,8 mm beträgt.

12. Kit (1) zahnärztlicher Instrumente gemäß einem der vorangehenden Ansprüche, wobei das erste Instrument für den linken Unterkiefer des Patienten und das zweite Instrument für den rechten Unterkiefer des Patienten eine Gesamtlänge zwischen 150 mm und 155 mm aufweisen; der linke seitliche Arm oder der rechte seitliche Arm der Instrumente für den Unterkiefer des Patienten (10, 100, 1000) eine Länge zwischen 55 und 71 mm aufweist; der vordere Arm (30, 300, 3000) für die linke oder rechte Seite eine Länge zwischen 62 und 70 mm aufweist; die Arme des Instruments für den Oberkiefer (50, 500, 5000) eine Länge zwischen 45 und 60 mm aufweisen und der Abstand zwischen ihren freien Enden zwischen 55 und 70 mm beträgt.

13. Kit (1) zahnärztlicher Instrumente gemäß dem vorangehenden Anspruch, **wobei** der linke seitliche Arm oder der rechte seitliche Arm der Instrumente für den Unterkiefer des Patienten (10, 100, 1000) eine Länge von 63 mm aufweist; der vordere Arm (30, 300, 3000) für die linke Seite oder die rechte Seite eine Länge von 66 mm aufweist; die Arme des Instruments für den Oberkiefer (50, 500, 5000) eine Länge von 55 mm aufweisen und der Abstand zwischen ihren freien Enden 65 mm beträgt.

## Revendications

1. Kit (1) d'instruments dentaires pour numérisation intraorale, ledit kit comprenant au moins un instrument pour l'arcade inférieure gauche (10, 100, 1000) du patient, au moins un instrument pour l'arcade inférieure droite du patient, ce dernier étant structurellement en miroir par rapport audit instrument pour l'arcade inférieure gauche du patient, au moins un instrument pour l'arcade supérieure (50, 500, 5000) du patient, lesdits instruments ayant chacun une poignée et deux bras, lesdits instruments pour l'arcade inférieure ayant chacun un bras courbé dirigé dans la direction latérale par rapport à la poignée et un bras courbé dirigé dans la direction avant, ledit instrument pour l'arcade supérieure ayant les bras avec l'un configuré dans la direction latérale droite et l'autre dans la direction latérale gauche, lesdits bras latéraux étant adaptés pour ouvrir les joues largement et permettre le glissement d'un scanner sur leur corps, ledit kit (1) d'instruments dentaires étant **caractérisé en ce que** chacun desdits instruments qui y sont compris est représenté par un seul corps, c'est-à-dire un monobloc.

2. Kit (1) d'instruments dentaires selon la revendication précédente dans lequel au moins un instrument est compris pour l'arcade inférieure gauche (10) du patient, ainsi que sa contrepartie en miroir, ce dernier étant au moins un instrument pour l'arcade inférieure droite du patient ; et au moins un instrument pour l'arcade supérieure (50) du patient est également compris, ledit instrument pour l'arcade inférieure gauche (10) du patient et respectivement sa contrepartie en miroir pour l'arcade inférieure droite du patient comprenant chacun une première poignée inférieure (20), un premier bras inférieur avant (30) et un premier bras inférieur latéral gauche (40) et respectivement un bras inférieur latéral droit, ledit instrument pour l'arcade supérieure (50) du patient comprenant : une première poignée supérieure (60) et deux bras latéraux supérieurs (70), ledit instrument pour l'arcade inférieure gauche (10) du patient, et l'arcade inférieure droite du patient, et ledit instrument pour l'arcade supérieure (50) du patient étant tels que leurs poignées, c'est-à-dire lesdites première poignée inférieure (20) et première poignée supérieure (60), présentent un profil en forme de parallélépipède à bords arrondis, lesdits instruments du kit étant chacun représentés par un seul corps, c'est-à-dire un monobloc, dans lequel les bras et la poignée sont représentés par un monobloc de matériau polymère.

3. Kit (1) d'instruments dentaires selon la revendication 1, dans lequel au moins un instrument pour l'arcade inférieure gauche (10) du patient est compris ainsi que sa contrepartie en miroir, ce dernier étant au moins un instrument pour l'arcade inférieure droite du patient ; et au moins un instrument pour l'arcade supérieure (50) du patient, ledit instrument pour l'arcade inférieure gauche (10) du patient et respectivement sa contrepartie en miroir pour l'arcade inférieure droite du patient comprenant chacun une première poignée inférieure (20), un premier bras inférieur avant (30) et un premier bras inférieur latéral gauche (40) et respectivement latéral droit, ledit instrument pour l'arcade supérieure (50) du patient comprenant : une première poignée supérieure (60) et deux bras latéraux supérieurs (70), ledit instrument pour l'arcade inférieure gauche (10) du patient, et l'arcade inférieure droite du patient, et ledit instrument pour l'arcade supérieure (50) du patient étant tels que leurs poignées, c'est-à-dire lesdites première poignée inférieure (20) et première poignée supérieure (60), présentent un profil en forme de parallélépipède à bords arrondis, lesdits instruments du kit étant chacun représentés par un seul corps, c'est-à-dire un monobloc, dans lequel les bras et la poignée sont assemblés par soudage.

4. Kit (1) d'instruments dentaires selon la revendication 1 dans lequel au moins un instrument pour l'arcade inférieure gauche (100) du patient est compris ainsi que sa contrepartie en miroir, c'est-à-dire au moins un instrument pour l'arcade inférieure droite du patient ; et au moins un instrument pour l'arcade supérieure (500) du patient, ledit instrument pour l'arcade inférieure gauche (100) du patient et respectivement sa contrepartie en miroir pour l'arcade inférieure droite comprenant une première poignée inférieure (200), un premier bras inférieur avant (300) et un premier bras inférieur latéral gauche (400) et respectivement latéral droit, ledit instrument pour l'arcade supérieure (500) du patient comprenant une première poignée supérieure (600) et deux bras latéraux supérieurs (700), lesdits instruments pour l'arcade inférieure gauche et droite (100) du patient et ledit instrument pour l'arcade supérieure (500) étant représentés par une seule pièce, c'est-à-dire un monobloc, de plaque courbée, plein ou perforé.

5. Kit (1) d'instruments dentaires selon la revendication précédente **dans lequel** la plaque est en métal, ladite plaque ayant une épaisseur comprise entre 0,5 mm et 1,2 mm.

6. Kit (1) d'instruments dentaires selon la revendication précédente **dans lequel** la plaque a une épaisseur de 0,8 mm.

7. Kit (1) selon l'une des revendications 4 et 6 **dans lequel** la plaque est selon le souhait en titane ou en acier.

8. Kit (1) d'instruments dentaires selon la revendication 1 **dans lequel** au moins un instrument pour l'arcade inférieure gauche (1000) du patient est compris ainsi que sa contrepartie en miroir, à savoir au moins un instrument pour l'arcade inférieure droite du patient ; et au moins un instrument pour l'arcade supérieure (5000) du patient est également compris, ledit instrument pour l'arcade inférieure gauche (1000) du patient, et respectivement sa contrepartie en miroir pour le côté droit, comprenant une première poignée inférieure (2000), un premier bras inférieur avant (3000) et un premier bras inférieur latéral gauche (4000) et respectivement latéral droit, ledit instrument pour l'arcade supérieure (5000) du patient comprenant une première poignée supérieure (6000) et deux bras latéraux supérieurs (7000), chacun desdits instruments pour l'arcade inférieure gauche (1000) et celui pour l'arcade inférieure droite du patient et l'instrument pour l'arcade supérieure (5000) du patient étant représenté par un seul fil en matériau métallique et/ou polymère.

9. Kit (1) d'instruments dentaires selon la revendication précédente **dans lequel** les instruments du kit sont en titane ou en acier.

10. Kit (1) d'instruments dentaires selon l'une des revendications 8 et 9 **dans lequel** l'épaisseur du fil avec lequel les instruments du kit sont obtenus est comprise entre 2 mm et 5 mm.

11. Kit (1) d'instruments dentaires selon la revendication précédente **dans lequel** l'épaisseur avec laquelle les instruments dudit kit sont atteints est de 3,8 mm.

12. Kit (1) d'instruments dentaires selon l'une des revendications précédentes dans lequel ledit premier instrument pour l'arcade inférieure gauche du patient et le deuxième instrument pour l'arcade inférieure droite du patient ont une longueur totale comprise entre 150 mm et 155 mm ; le bras latéral gauche, ou bras latéral droit des instruments pour l'arcade inférieure du patient (10, 100, 1000) ont une longueur comprise entre 55 et 71 mm ; le bras avant (30, 300, 3000) pour le côté gauche ou côté droit a une longueur comprise entre 62 et 70 mm ; les bras de l'instrument pour l'arcade supérieure (50, 500, 5000) ont une longueur comprise entre 45 et 60 mm, et la distance entre leur extrémité libre est comprise entre 55 et 70 mm.

13. Kit (1) d'instruments dentaires selon la revendication précédente **dans lequel** le bras latéral gauche, ou bras latéral droit des instruments pour l'arcade inférieure du patient (10, 100, 1000) ont une longueur de 63 mm ; le bras avant (30, 300, 3000) pour le côté gauche ou côté droit ont une longueur de 66 mm ; les bras de l'instrument pour l'arcade supérieure (50, 500, 5000) ont une longueur de 55 mm, et la distance entre leurs extrémités libres est de 65 mm.
